# EUROPÄISCHE PATENTSCHRIFT

(11) **EP 0 393 608 B1**
(45) Veröffentlichungstag und Bekanntmachung des Hinweises auf die Patenterteilung: **28.06.1995**
(21) Anmeldenummer: 90107303.1
(22) Anmeldetag: 18.04.1990
(51) Int. Cl.: A61F 2/36

(54) **Oberschenkelteil einer Hüftgelenk-Endoprothese**
Femoral headpart of a hip joint endoprosthesis
Tête fémorale d'une endoprothèse pour l'articulation de la hanche

(30) Priorität: 19.04.1989 DE 3912753
(43) Veröffentlichungstag der Anmeldung: 24.10.1990
(73) Patentinhaber: Möbius, Joachim, Dr., D-58452 Witten (DE)
(72) Erfinder: Möbius, Joachim, Dr., D-58452 Witten (DE)
(74) Vertreter: Köchling, Conrad-Joachim

(56) Entgegenhaltungen:
- DE-A- 3 415 934
- DE-A- 3 528 151
- DE-A- 3 737 372

## Beschreibung

Die Erfindung betrifft ein Oberschenkelteil einer Hüftgelenk-Endoprothese für zementlose Verankerung, bestehend aus einem im natürlichen (Markraum) oder gebohrten Knochenkanal verankerbaren Schaft und einem auf den Schaft aufsteckbaren Gelenkkopf mit Gelenkkugel, Hals und verbreiterter gegenüber dem Schaft geneigter Fußplatte, wobei der mit Außengewinde versehene Schaft in den Knochenkanal einschraubbar ist und der Gelenkkopf auf das obere dem Anschlußteil des Gelenkkopfes angepaßte Ende aufschiebbar und mittels einer Arretierschraube lagefixierbar ist.

Aus dem Stand der Technik (DE-GM 77 23 852) ist ein Oberschenkelteil dieser Art bekannt, bei dem der ein Außengewinde aufweisende Schaft in den Knochenkanal eingeschraubt wird und anschließend der mit einer konischen Aufnahmebohrung versehene Gelenkkopf auf das freie, der konischen Aufnahmebohrung angepaßte Ende des Schaftes aufgesteckt wird. Der Gelenkkopf weist dabei eine Fußplatte auf, die auf die mittels einer Säge hergestellte obere Knochenschnittfläche möglichst flächenbündig aufgesetzt wird. Als Verdrehsicherung weist dabei die Fußplatte noch Annagelungsspitzen auf, die in die Knochenschnittfläche eindringen sollen.

Bei dieser Ausbildung tritt das Problem auf, daß eine exakte Anlage der Fußplatte an die Knochenschnittfläche nur rein zufällig erreicht wird. Die Knochenschnittfläche wird üblicherweise durch eine Säge erzeugt, wobei die Resektionsebene schräg steht. Wird nun der Schaft in den Knochenkanal eingeschraubt und der Gelenkkopf auf den Schaft aufgesteckt, so ist nur ausnahmsweise eine genaue Anlage der Unterfläche der Fußplatte an die Resektionsebene des Knochens möglich. Die Relativlage von Gelenkkopf und Schaft ist durch die entsprechenden konischen Ausbildungen der Kontaktflächen zwangsweise auf eine bestimmte Lage festgelegt. Sofern die relative Drehlage des Schaftes nicht genau passend eingestellt ist, so ergibt sich eine einseitige Anlage der Fußplatte an die Knochenschnittfläche. Durch die unterschiedliche Druckbelastung des Knochens in der Schnittfläche kann eine Schrumpfung des Knochens erfolgen, dem vorzubeugen ist. Desweiteren ist im Stand der Technik die Anordnung von Annagelungsspitzen notwendig, die in das Knochenmaterial eingetrieben werden müssen, um eine Sicherung gegen relative Verdrehung bei der Benutzung der Prothese sicherzustellen. Jedoch ist auch diese Lösung insofern nicht günstig, als durch die Annagelungsspitzen in Verbindung mit einer nicht exakten Anpassung der Fußplatte an die Resektionsebene des Knochens ein gewisses Bewegungsspiel zwangsläufig über eine längere Benutzungsdauer entsteht.

Ausgehend von diesem Stand der Technik liegt der Erfindung die Aufgabe zugrunde, eine Endoprothese gattungsgemäßer Art zu schaffen, bei der eine exakte Anlage der Fußplatte an die Resektionsebene des Knochens erreichbar ist.

Zur Lösung dieser Aufgabe wird vorgeschlagen, daß der Schaft an seinem dem Gelenkkopf zugewandten Ende eine quer zur Schaftachse gerichtete Stirnfläche mit Radial-Verzahnung aufweist,
von der Fußplatte des Gelenkkopfes eine hülsenartige Aufnahme für das Schaftende abragt, die in ihrer Basis eine zur Stirnflächen-Verzahnung passende Radial-Verzahnung aufweist, und
daß die Fußplatte auf ihrer dem Schaft zugewandten Seite ebenflächig ausgebildet ist.

Diese Ausbildung ermöglicht es, daß nach dem Sägen der relativ schrägstehenden Resektionsebene der Schaft in den Knochenkanal nach Vorschneiden eines Knochengewindeganges eingeschraubt wird und dann der Gelenkkopf exakt lagegerecht auf das freie Ende des Schaftes aufgesteckt wird, so daß eine exakte oder nahezu exakte Anlage der ebenen Fläche der Fußplatte an die Knochenschnittfläche erfolgen kann. Es ist lediglich vom Grad der Zahnteilung der Genauigkeitsgrad abhängig. Nach ordnungsgemäßer Ausrichtung des Gelenkkopfes wird dann die Arretierschraube durch eine entsprechende Öffnung der Fußplatte hindurchgesteckt und in die Gewindebohrung des Schaftteiles eingeschraubt. Eine relative Drehung von Schaft zu Gelenkkopf ist dann durch die ineinandergreifende Verzahnung von hülsenartiger Aufnahme und Schaft ausgeschlossen. Durch die exakte Ausrichtung der Fußplatte relativ zu der Knochenschnittfläche ist erreicht, daß alle auftretenden Belastungskräfte großflächig und gleichmäßig von dem Prothesenteil in den Knochen eingeleitet werden. Infolge der Tatsache, daß die Knochenschnittfläche schräg zur Knochenschaftlängsachse gerichtet ist und die Fußplatte in gleicher Weise zur Prothesenschaftlängsachse ausgerichtet ist, ist sichergestellt, daß eine hohe Sicherheit gegen Verdrehung gegeben ist. Beim Einsatz der entsprechenden Prothese ist bereits nach vollendeter Operation eine sehr stabile Verbindung mit dem Knochen geschaffen, die eine frühzeitige Belastung und eine dauerhafte sichere Halterung gewährleistet.

In Weiterbildung wird vorgeschlagen, daß zwischen der ebenen unteren Fläche der Fußplatte und ihrer hülsenartigen Aufnahme mindestens eine Rippe mit zur Schaftlängserstreckung paralleler Erstreckung ausgebildet ist.

Durch diese Rippe wird eine zusätzliche Sicherung gegen Verdrehungsmomente gebildet, ohne daß hierdurch die Funktion der Prothese im übrigen beeinträchtigt wäre.

Infolge der Tatsache, daß der Knochenkanal der zur Aufnahme des Schaftes dient, normalerweise unsymmetrisch zur elliptischen Resektionsebene liegt, ist erfindungsgemäß vorgesehen, daß die hülsenartige Aufnahme und der Hals asymmetrisch -sowohl in Längs- als auch in Querrichtung der Fußplatte gesehen- zu dieser versetzt angeformt sind.

Es ist zwar bei dieser Ausbildung notwendig, daß der Gelenkkopf für den linken Oberschenkel anders ausgebildet ist, als für den rechten Oberschenkel, jedoch wird für die asymmetrische Ausbildung sichergestellt, daß die Fußplatte ganzflächig auf der Knochenschnittfläche aufliegen kann.

Weiterhin ist vorteilhaft, daß die Fußplatte neben dem Hals eine die Fußplatte durchgreifende Lochung aufweist.

Durch diese Lochung kann unter Druck eine selektive Zementierung des verbliebenen Hohlraumes unterhalb der Fußplatte erfolgen, sofern dies für notwendig erachtet wird. Dabei erscheint wesentlich, daß diese Zementierung nur in dem äußersten oberen Bereich des Hohlraumes erfolgt, nicht aber in der Gewindezone des Schaftes, so daß falls später einmal die Prothese entfernt werden müßte, diese Entfernung leicht vorgenommen werden kann, indem der Gelenkkopf abgenommen und die Zementierung aus der leicht zugänglichen Höhlung des Knochens entfernt wird. Auch der Schaft ist entsprechend aus dem Knochenkanal herausschraubbar.

Bevorzugt ist ferner vorgesehen, daß die Lochung in der Ebene angeordnet ist, die von der Halsmittelachse und der Aufnahmemittelachse aufgespannt ist, und zwar in dem von den beiden Achsen eingeschlossenen Winkelbereich.

Weiterhin ist vorteilhaft, daß die Lochung unmittelbar neben der Stützrippe angeordnet ist und die Stützrippe eine weitere Lochung aufweist.

Dabei ist vorzugsweise vorgesehen, daß die weitere Lochung am Ende des durch die erste Lochung gebildeten Kanals ausgebildet ist.

Durch diese Ausbildung ist es möglich mit einer geeigneten Spritze oder dergleichen sehr leicht beidseits der Stützrippe eine Zementierung vorzunehmen.

Weiterhin ist vorzugsweise vorgesehen, daß der Schaft für das linke Oberschenkelteil Rechtsgewinde und für das rechte Linksgewinde aufweist.

Diese Anordnung wird deswegen als vorteilhaft angesehen, weil entsprechend den natürlichen Bewegungsabläufen bei der Bewegung des Oberschenkels unterschiedliche Drehmomente auf die Prothese einwirken, so daß eine unterschiedliche Gewindeausbildung für das rechte und linke Oberschenkelteil der unerwünschten Verdrehung weiter entgegenwirkt.

Eine besonders bevorzugte Weiterbildung wird darin gesehen, daß ein Fräsdorn vorgesehen ist, der auf das freie Ende des Schaftes aufsteckbar und dort befestigbar ist und dessen der Radial-Verzahnung des Schaftes zugewandte Fläche ebenfalls Radial-Verzahnung aufweist.

Durch diese Anordnung ist es möglich, eine besonders exakte Anpassung der schrägstehenden Resektionsebene an die Anlagefläche der Fußplatte zu erreichen. Beim Einsatz der Prothese wird zunächst der Schaft in den natürlichen oder vorgebohrten Knochenkanal eingeschraubt. Auf das freie Ende des Schaftes wird dann der Fräsdorn aufgesteckt und in dieser Lage arretiert, wobei die Radial-Verzahnung von Fräsdorn und Schaft ineinandergreifen, so daß beide Teile relativ zueinander unverdrehbar angeordnet sind. Es kann dann mit einer sogenannten Calcar-Fräse die schrägstehende Resektionsebene erzeugt werden. Sobald diese elliptische Knochenschnittfläche gebildet ist, kann die Fräse entfernt und der Fräsdorn vom freien Ende des Schaftes gelöst werden. Nachfolgend kann dann der Gelenkkopf mit seiner Hülse über das freie Schaftende geschoben werden, bis die Verzahnungen der Aufnahme und des Schaftendes ineinandergreifen. Dabei ist leicht die entsprechend gleiche Relativlage der Zähne zueinander einzustellen, die dem Fräsvorgang entspricht. Auf diese Weise ist exakt genau die Position erreicht, in der die ebene Fußplattenanlagefläche vollflächig auf der Knochenschnittfläche aufliegt.

Es hat sich ferner als vorteilhaft erwiesen, daß die Radial-Verzahnungen dreißig Zähne aufweisen.

Durch diese Ausbildung ist erreicht, daß ein Versatz um einen Zahn einen Winkelversatz von etwa 12° entspricht. Diese Versatzmöglichkeit reicht aus, damit ohne Verdrehung des Prothesenschaftes der Fräsdorn winkelgerecht entsprechend dem Antetorsionswinkel des Schenkelhalses ausgerichtet werden kann.

Zur Erzeugung der Knocheneinschnitte, in welche die Rippen beim Aufschieben der Fußplatte auf den Schaft eingleiten, ist vorgesehen, daß eine Sägeschablone vorgesehen ist, die auf das Ende des Schaftes aufsteckbar und dort befestigbar ist und deren der Radialverzahnung des Schaftes zugewandte Fläche ebenfalls Radial-Verzahnung aufweist, wobei die Sägeschablone eine der Fußplatte entsprechende Platte umfaßt, die mindestens einen Schlitz als Führung für eine Säge aufweist, der an der Stelle der Platte angeordnet ist, der dem Ort der Anordnung der Rippe an der Fußplatte entspricht.

Vorzugsweise ist dabei vorgesehen, daß die Platte nur etwa im relativ unteren Schrägbereich ausgebildet ist, der dem Fußplattenbereich zwischen der Rippe und der dieser diametral gegenüber befindlichen Stelle entspricht, wobei die Randkanten der Teilplatte parallel zur Mittellängsachse des Schaftes gerichtet verlaufen und mit der Flanke der Rippe der Fußplatte fluchten, so daß eine der Randkanten als Stützfläche für eine Säge wirkt, die zur Erzeugung des Aufnahmeschlitzes für die Rippe parallel zur Schaftachse an der Randkante vorbeigeführt wird.

Die Sägeschablone weist zudem eine Führungshülse auf, die in der Sollposition über das freie Ende des Schaftes greift. Die Lage wird durch eine entsprechende Schraube gesichert. (Vergleiche Schraubbefestigung des Gelenkkopfes. ) Durch die gleiche Ausrichtung der Schablone wie sie der Fußplatte entspricht und durch die Anordnung der Verzahnung kann die Schablone nach der Erzeugung der Resektionsebene auf den Schaft gesteckt und mittels der Schraube gesichert werden. Nachfolgend kann mit einer Säge, die in den randoffenen, relativ untenliegenden Radialschlitz eingeführt wird, der entsprechende Aufnahmeschlitz am Knochen für die Rippe geschaffen werden. Der für die seitliche weitere Rippe notwendige Schlitz kann erzeugt werden, indem die Säge an der seitlichen Flanke der Platte der Schablone vorbeigeführt wird.

Die Erfindung ist nachstehend anhand eines Ausführungsbeispieles erläutert.

Es zeigt:
- Fig. 1: Das Oberschenkelteil einer Hüftgelenk-Endoprothese in der Gebrauchsstellung;
- Fig. 2: eine Einzelheit in Vorderansicht;
- Fig. 3: die Einzelheit von der Rückseite;
- Fig. 4: eine Einzelheit in Ansicht.

Das Oberschenkelteil einer Hüftgelenk-Endoprothese für zementlose Verankerung besteht im wesentlichen aus einem im Knochenkanal 1 des Oberschenkelknochens 2 verankerbaren Schaft 3 und einem auf den Schaft aufsteckbaren Gelenkkopf 4 mit Gelenkkugel 5, Hals 6 und Fußplatte 7.

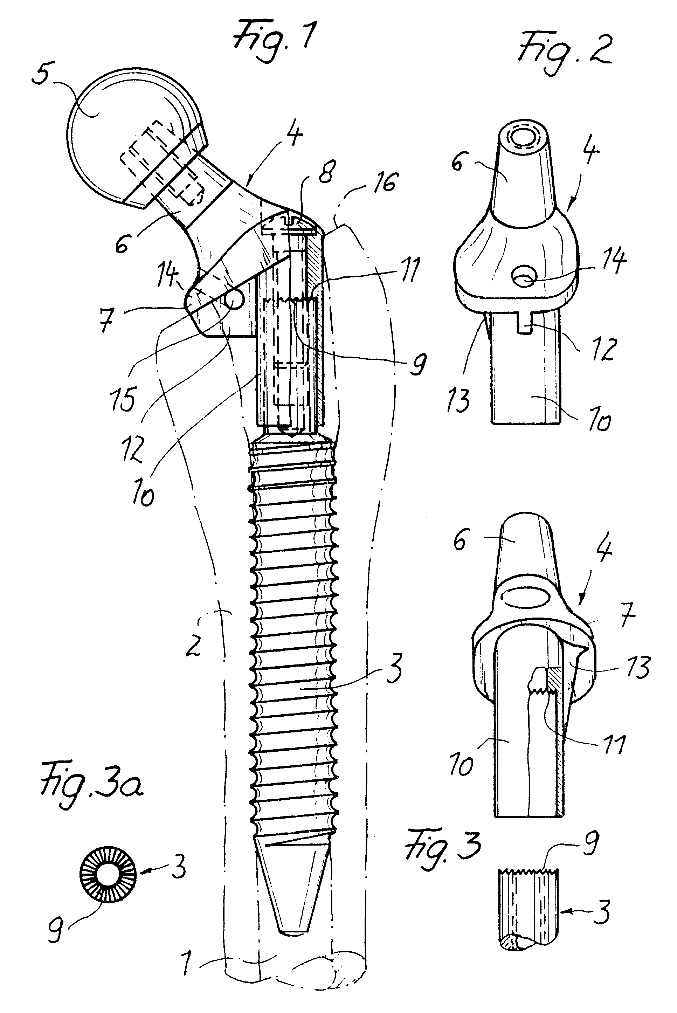

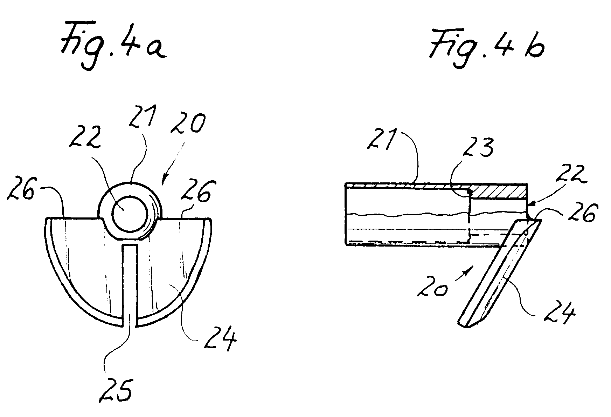

Der mit Außengewinde versehene Schaft 3 ist in den Knochenkanal 1 eingeschraubt und auf das obere Ende des Schaftes 3 ist der Gelenkkopf 4 aufgeschoben und mittels einer Arretierschraube 8 lagegesichert. Der Schaft 3 weist an seinem dem Gelenkkopf 4 zugewandten Ende eine quer, insbesondere rechtwinklig zur Schaftachse gerichtete Stirnfläche mit Radial-Verzahnung 9 auf, wie dies insbesondere aus den Figuren 3 und 3a ersichtlich ist. Von der Fußplatte 7 des Gelenkkopfes 4 ragt eine hülsenartige Aufnahme 10 für das gegenüber dem Gewindeteil im Durchmesser kleinere Schaftende ab. Die hülsenartige Aufnahme 10 ist ebenso wie das freie Ende des Schaftes 3 zylindrisch ausgebildet und im Querschnitt kreisrund. Die Aufnahme 10 weist in ihrer Basis eine zur Stirnflächenverzahnung (Radial-Verzahnung 9) passende Radial-Verzahnung 11 auf. Die Fußplatte ist auf ihrer dem Schaft 3 zugewandten Seite im wesentlichen ebenflächig ausgebildet. Zwischen der ebenen unteren Fläche der Fußplatte 7 und ihrer hülsenartigen Aufnahme 10 ist eine relativ große Rippe 12 in dem Bereich des eingeschlossenen Winkels zwischen Schaft 3 und Gelenkkopf 4 angeordnet und zusätzlich eine weitere kleine Rippe 13 quer dazu verlaufend um 90° versetzt an der größeren Erstreckung der Fußplatte 7. Die Rippen sind sowohl an der Fußplatte 7 als auch an dem Aufnahmeteil 10 angeformt. Beide Rippen 12, 13 erstrecken sich parallel zur Schaftlängserstreckung. Die hülsenartige Aufnahme 10 und der Hals 6 sind sowohl in Längs- als auch in Querrichtung asymmetrisch relativ zur Fußplatte 7 versetzt angeformt, was den entsprechenden Verhältnissen an der Schnittfläche des Oberschenkelknochens entspricht. Aufgrund dieser Ausbildung ist eine Ausführungsform für den linken Oberschenkel (in der Zeichnung dargestellt) und eine spiegelsymmetrisch hierzu ausgebildete Ausführungsform für den rechten Oberschenkel notwendig. Zusätzlich weist die Fußplatte 7 neben dem Hals 6 eine die Fußplatte durchgreifende Lochung 14 auf. Die Lochung ist in der Ebene angeordnet, die von der Halsmittelachse und der Aufnahmemittelachse aufgespannt ist, und zwar in dem von den beiden Achsen eingeschlossenen Winkelbereich. Desweiteren ist die Lochung unmittelbar oberhalb oder leicht seitlich versetzt zur Stützrippe 12 angeordnet, wobei die Rippe eine weitere Lochung 15 aufweist, so daß gegebenenfalls durch die Lochung 14 eingespritzte Zementierungsmasse in die Hohlräume dringen kann, die beidseits der Rippe 12 im Oberschenkelknochen gebildet sind.

Der Schaft 3 weist im Ausführungsbeispiel Rechtsgewinde auf und ist für das linke Oberschenkelteil vorgesehen, während der für das rechte Oberschenkelteil vorgesehene Schaft 3 Linksgewinde aufweisen soll. Zur Herstellung der elliptischen Resektionsebene 16 des Oberschenkelknochens 2 kann auf das freie Ende des Schaftes 3 nach dessen Anordnung in dem Knochenkanal 1 ein nicht dargestellter Fräsdorn aufgeschoben werden, der eine zur Radial-Verzahnung 9 passende Verzahnung an der Kontaktfläche aufweist und mit dem Schaft beispielsweise mittels einer Schraube fest verbindbar ist. Der Fräsdorn sollte gleich abgewinkelt sein, wie dies der Ausbildung des Kupplungsteiles 4 (Hülsenteil 10, Hals 6) entspricht. Der Fräsdorn ist in entsprechender richtiger Lage anzuordnen, so daß die gewünschte Resektionsebene 16 erzeugbar ist. Das Fräswerkzeug kann um den Fräsdorn umlaufen und die entsprechende Resektionsebene so hergestellt werden. Anschließend kann der Fräsdorn vom Schaft 3 gelöst werden und der Gelenkkopf so auf den Schaft 3 aufgesteckt werden, daß exakt die gleiche Lage eingestellt ist, wie sie der Fräsdorn vorher hatte. Auf diese Weise wird erreicht, daß die Anlagefläche der Fußplatte 7 exakt planparallel zur Resektionsebene anzuordnen ist. Um eine ausreichende relative Winkelverstellung insbesondere des Fräsdornes zum Erzeugen der Resektionsebene 16 zu ermöglichen, soll die Radial-Verzahnung 9 und die dazu passenden Verzahnungen von Aufnahme 10 bzw. Fräsdorn dreißig Zähne aufweisen, so daß eine 12°-Teilung erreicht ist. Durch die 12°-Teilung beider zusammenwirkender Elemente wird eine Verstellmöglichkeit im 6°-Bereich erreicht. Diese Verstellmöglichkeit reicht regelmäßig aus.

Zur Erzeugung der Aufnahmeschlitze an der Knochenstirnseite für die Rippen 12, 13 ist eine Sägeschablone 20 vorgesehen. (Vergleiche Figur 4). Diese weist eine Hülse 21 mit einer Stufenbohrung 22 auf, die zur Anordnung einer Radial-Verzahnung 23 und zur Aufnahme der Schraube 8 dient. An der Hülse 21 ist eine Platte 24 befestigt (unlösbar), die gleiche Neigung relativ zur Schaftachse bzw. Hülsenachse aufweist wie die Fußplatte 7. An der Stelle an welcher die Fußplatte 7 die Rippe 12 besitzt, ist an der Platte 24 ein randoffener Radial-Schlitz 25 ausgebildet, durch den eine Säge oder ein vergleichbares Werkzeug geführt ist, wenn die schlitzförmige Aufnahme für die Rippe 12 am Knochenrand vorgesehen wird.

Im Ausführungsbeispiel erstreckt sich die Platte 24 nur über den Bereich der bei der Fußplatte 7 von seitlich der Rippe 13 (relativ untere Flanke gemäß Figur 2) bis diametral gegenüber und lediglich im unteren Bereich gemäß Figur 2. Durch die so geschaffenen Randkanten 26 ist eine Führung für eine Säge oder dergleichen gebildet, die zur Herstellung eines Einsatzschlitzes für die Rippe 13 am Knochen dient.

## Patentansprüche

1. Oberschenkelteil einer Hüftgelenk-Endoprothese für zementlose Verankerung, bestehend aus einem im natürlichen (Markraum) oder gebohrten Knochenkanal verankerbaren Schaft und einem auf den Schaft aufsteckbaren Gelenkkopf, mit Gelenkkugel, Hals und verbreiterter gegenüber dem Schaft geneigter Fußplatte, wobei der mit Außengewinde versehene Schaft in den Knochenkanal einschraubbar ist und der Gelenkkopf auf das obere Anschlußteil des Gelenkkopfes angepaßte Ende aufschiebbar und mittels einer Arretierschraube lagefixierbar ist, **dadurch gekennzeichnet, daß**
der Schaft (3) an seinem dem Gelenkkopf (4) zugewandten Ende eine quer zur Schaftachse gerichtete Stirnfläche mit Radial-Verzahnung (9) aufweist,
von der Fußplatte (7) des Gelenkkopfes (4) eine hülsenartige Aufnahme (10) für das Schaftende abragt, die in ihrer Basis eine zur Stirnflächen-Verzahnung passende Radial-Verzahnung (11) aufweist, und
daß die Fußplatte (7) auf ihrer dem Schaft (3) zugewandten Seite ebenflächig ausgebildet ist.

2. Oberschenkelteil nach Anspruch 1, **dadurch gekennzeichnet, daß** das zwischen der ebenen unteren Fläche der Fußplatte (7) und ihrer hülsenartigen Aufnahme (10) mindestens eine Rippe (12, 13) mit zur Schaftlängserstreckung paralleler Erstreckung ausgebildet ist.

3. Oberschenkelteil nach Anspruch 1 oder 2, **dadurch gekennzeichnet, daß** die hülsenartige Aufnahme (10) und der Hals (6) asymmetrisch -sowohl in Längs-als auch in Querrichtung der Fußplatte (7) gesehen- zu dieser versetzt angeformt sind.

4. Oberschenkelteil nach einem der Ansprüche 1 bis 3, **dadurch gekennzeichnet, daß** die Fußplatte (7) neben dem Hals (6) eine die Fußplatte (7) durchgreifende Lochung (14) aufweist.

5. Oberschenkelteil nach Anspruch 4, **dadurch gekennzeichnet, daß** die Lochung (14) in der Ebene angeordnet ist, die von der Halsmittelachse und der Aufnahmemittelachse aufgespannt ist, und zwar in dem von den beiden Achsen eingeschlossenen Winkelbereich.

6. Oberschenkelteil nach Anspruch 4 oder 5, **dadurch gekennzeichnet, daß** die Lochung (14) unmittelbar neben der Stützrippe (12) angeordnet ist und die Stützrippe (12) eine weitere Lochung (15) aufweist.

7. Oberschenkelteil nach Anspruch 6, **dadurch gekennzeichnet, daß** die weitere Lochung (15) am Ende des durch die erste Lochung (14) gebildeten Kanals ausgebildet ist.

8. Oberschenkelteil nach einem der Ansprüche 1 bis 7, **dadurch gekennzeichnet, daß** der Schaft (3) für das linke Oberschenkelteil Rechtsgewinde und für das rechte Linksgewinde aufweist.

9. Oberschenkelteil nach einem der Ansprüche 1 bis 8, **dadurch gekennzeichnet, daß** ein Fräsdorn vorgesehen ist, der auf das freie Ende des Schaftes (3) aufsteckbar und dort befestigbar ist und dessen der Radial-Verzahnung (9) des Schaftes (3) zugewandte Fläche ebenfalls Radial-Verzahnung aufweist.

10. Oberschenkelteil nach einem der Ansprüche 1 bis 9, **ddurch gekennzeichnet, daß** die Radial-Verzahnungen (9, 10) dreißig Zähne aufweisen.

11. Oberschenkelteil nach einem der Ansprüche 1 bis 10, **dadurch gekennzeichnet, daß** eine Sägeschablone (20) vorgesehen ist, die auf das Ende des Schaftes (3) aufsteckbar und dort befestigbar ist und deren der Radialverzahnung (9) des Schaftes (3) zugewandte Fläche ebenfalls Radial-Verzahnung (23) aufweist, wobei die Sägeschablone (20) eine der Fußplatte (7) entsprechende Platte (24) umfaßt, die mindestens einen Schlitz (25) als Führung in eine Säge aufweist, der an der Stelle der Platte (24) angeordnet ist, der dem Ort der Anordnung der Rippe (12, 13) an der Fußplatte (7) entspricht.

12. Oberschenkelteil nach Anspruch 11, **dadurch gekennzeichnet, daß** die Platte (24) nur etwa im relativ unteren Schrägbereich ausgebildet ist, der dem Fußplattenbereich zwischen der Rippe (13) und der dieser diametral gegenüber befindlichen Stelle entspricht, wobei die Randkanten (26) der Teilplatte (24) parallel zur Mittellängsachse des Schaftes (3) gerichtet verlaufen und mit der Flanke der Rippe (13) der Fußplatte (7) fluchten, so daß eine der Randkanten (26) als Stützfläche für eine Säge wirkt, die zur Erzeugung des Aufnahmeschlitzes für die Rippe (13) parallel zur Schaftachse an der Randkante (26) vorbeigeführt wird.

## Claims

1. A femoral part of a hip joint endoprothesis for attachment without the use of cement, consisting of a shaft which can be attached in the natural (medullary space) or bored bone channel and an articular head which can be placed on the shaft, with a spherical part, neck and widened bearing plate inclined in relation to the shaft, wherein the shaft Provided with outer thread is Screwable into the bone channel and the articular head is Slidable onto the upper end which is adapted to the connecting part of the articular head and fixable in position by means of a locking screw, **characterised in that**
at its end close to the articular head (4) the shaft (3) has an end face with radial toothing (9) directed transversally to the shaft axis,
a sleeve-shaped housing (10) for the shaft end, which in its base comprises radial toothing (11) matching the toothing of the end face, protrudes from the bearing plate (7) of the articular head (4),
**and in that** the bearing plate (7) has a plane construction on its side close to the shaft (3).

2. A femoral part according to Claim 1,
**characterised in that** at least one rib (12, 13) having an extension parallel to the longitudinal extension of the shaft is constructed between the plane lower face of the bearing plate (7) and its sleeve-shaped housing (10).

3. A femoral part according to Claim 1 or 2,
**characterised in that**, seen both in the longitudinal and also in the transverse direction of the bearing plate (7), the sleeve-shaped housing (10) and the neck (6) are formed asymmetrically offset to said plate.

4. A femoral part according to one of Claims 1 to 3,
**characterised in that** the bearing plate (7) has a hole (14) passing through the bearing plate (7) next to the neck (6).

5. A femoral part according to Claim 4,
**characterised in that** the hole (14) is provided in the plane which is determined by the central axis of the neck and the central axis of the housing, and in fact in the angular region enclosed by both axes.

6. A femoral part according to Claim 4 or 5,
**characterised in that** the hole (14) is provided directly next to the support rib (12) and the support rib (12) has a further hole (15).

7. A femoral part according to Claim 6,
**characterised in that** the further hole (15) is constructed at the end of the channel formed by the first hole (14).

8. A femoral part according to one of Claims 1 to 7,
**characterised in that** the shaft (3) for the left femoral part has a right-handed thread and for the right femoral part has a left-handed thread.

9. A femoral part according to one of Claims 1 to 8,
**characterised in that** a cutter arbour is provided, which can be placed on the free end of the shaft (3) and can be attached thereto and in which the face close to the radial toothing (9) of the shaft (3) also has radial toothing.

10. A femoral part according to one of Claims 1 to 9,
**characterised in that** the radial toothing (9, 10) has thirty teeth.

11. A femoral part according to one of claims 1 to 10,
**characterised in that** a sawing template (20) is provided, which can be placed on the end of the shaft (3) and can be attached thereto, and whose face close to the radial toothing (9) of the shaft (3) also has radial toothing (23), the sawing template (20) surrounding a plate (24) corresponding to the bearing plate (7), which comprises at least one slit (25) as a guide for a saw, which is provided at the site in the plate (24) corresponding to the site of the provision of the rib (12,13) on the bearing plate (7).

12. A femoral part according to claim 11,
**characterised in that** the plate (24) is only constructed roughly in the relatively lower inclined region which corresponds to the bearing plate region between the rib (13) and the site diametrally opposite it, with the edges (26) of the plate (24) extending in a direction parallel to the central longitudinal axis of the shaft (3) and in alignment with the edge of the rib (13) on the bearing plate (7), so that one of the edges (26) acts as a support face for a saw which is guided past parallel to the shaft axis at the edge (26) to produce the slit for receiving the rib (13).

## Revendications

1. Partie fémorale d'une endoprothèse de la hanche destinée à un ancrage sans ciment, constituée par une tige qui peut être ancrée dans un canal osseux naturel (canal médullaire) ou ménagé par perçage et par une tête d'articulation qui peut être enfoncée sur la tige et qui comprend une rotule, un col et une plaque de base élargie et inclinée par rapport à la tige, cependant que la tige pourvue d'un filetage extérieur peut être vissée dans le canal médullaire, et que la tête d'articulation peut être enfoncée sur l'extrémité supérieure de la tige qui est adaptée à la partie de raccordement de la tête d'articulation, en pouvant être fixée en position au moyen d'une vis de blocage, caractérisée par le fait que la tige (3) présente, à son extrémité qui est tournée vers la tête d'articulation (4), une surface frontale dirigée transversalement par rapport à l'axe de la tige et pourvue d'une denture radiale (9), par le fait qu'un logement en forme de manchon (10) destiné à l'extrémité de la tige fait saillie depuis la plaque de base (7) de la tête d'articulation (4), et qu'il présente dans son fond une denture radiale (11) qui est adaptée à la denture de la surface frontale, et par le fait que la plaque de base (7) est réalisée sous la forme d'une surface plane sur son côté qui est tourné vers la tige (3).

2. Partie fémorale selon la revendication 1, caractérisée par le fait qu'au moins une nervure (12, 13) qui s'étend parallèlement à la direction longitudinale de la tige est formée entre la surface inférieure plane de la plaque de base (7) et son logement en forme de manchon (10).

3. Partie fémorale selon la revendication 1 ou 2, caractérisée par le fait que le logement en forme de manchon (10) et le col (6) sont formés en étant décalés d'une manière asymétrique par rapport à la plaque de base (7) - tant dans la direction horizontale de celle-ci que dans sa direction transversale.

4. Partie fémorale selon l'une des revendications 1 à 3, caractérisée par le fait que la plaque de base (7) présente à côté du col (6) un perçage (14) qui traverse la plaque de base (7).

5. Partie fémorale selon la revendication 4, caractérisée par le fait que le perçage (14) est disposé dans le plan qui passe par l'axe central du col et par l'axe central du logement, et ce, dans la zone angulaire formée par ces deux axes.

6. Partie fémorale selon la revendication 4 ou 5, caractérisée par le fait que le perçage (14) est disposé immédiatement à côté de la nervure d'appui (12), et que la nervure d'appui (12) présente un autre perçage (15).

7. Partie fémorale selon la revendication 6, caractérisée par le fait que l'autre perçage (15) est ménagé à l'extrémité du canal qui est formé par le premier perçage (14).

8. Partie fémorale selon l'une des revendications 1 à 7, caractérisée par le fait que la tige (3) qui est destinée à la partie fémorale gauche présente un filetage à droite et que celle destinée à la partie fémorale droite présente un filetage à gauche.

9. Partie fémorale selon l'une des revendications 1 à 8, caractérisée par le fait qu'il est prévu un mandrin de fraisage qui peut être enfoncé sur l'extrémité libre de la tige (3), qui peut être fixé à cet endroit et dont la surface tournée vers la denture radiale (9) de la tige (3) présente également une denture radiale.

10. Partie fémorale selon l'une des revendications 1 à 9, caractérisée par le fait que les dentures radiales (9, 11) comportent trente dents.

11. Partie fémorale selon l'une des revendications 1 à 10, caractérisée par le fait qu'il est prévu un guide-scie (20) qui peut être enfoncé sur l'extrémité de la tige (3), qui peut être fixé à cet endroit et dont la surface tournée vers la denture radiale (9) de la tige (3) présente également une denture radiale (23), cependant que le guide-scie (20) comprend une plaque (24) qui correspond à la plaque de base (7) et qui présente au moins une fente (25), celle-ci servant de guidage pour une soie et étant disposée à l'endroit de la plaque (24) qui correspond à l'endroit où la nervure (12, 13) est disposée sur la plaque de base (7).

12. Partie fémorale selon la revendication 11, caractérisée par le fait que la plaque (24) ne s'étend que sur une région oblique qui est une région inférieure par rapport à la région de la plaque de base correspondante qui est située entre la nervure (13) et l'endroit diamétralement opposé à celle-ci, cependant que les bords (26) de la plaque partielle (24) s'étendent en étant dirigés parallèlement à l'axe longitudinal central de la tige (3) et en étant alignés sur le flanc de la nervure (13) de la plaque de base (7), de sorte que l'un des bords (26) sert de surface d'appui à une soie qui est guidée sur le bord (26) parallèlement à l'axe de la tige afin d'engendrer la fente qui reçoit la nervure (13).
